# EUROPÄISCHE PATENTANMELDUNG

(11) **EP 1 273 285 A2**
(43) Veröffentlichungstag der Anmeldung: **08.01.2003**
(21) Anmeldenummer: 02014846.6
(22) Anmeldetag: 03.07.2002
(51) Int. Cl.: A61K 7/48

(54) **Verwendung von 5'-Desoxy-5'-methylthioadenosin in hautkosmetischen Mitteln**

(30) Priorität: 04.07.2001 DE 10132338
(71) Anmelder: BASF AKTIENGESELLSCHAFT, 67056 Ludwigshafen (DE)
(72) Erfinder: Jentzsch, Axel, 68167 Mannheim (DE); Kolter, Karl, 67117 Limburgerhof (DE); Sperling, Karin, 67433 Neustadt (DE)
(74) Vertreter: Kinzebach, Werner, Dr.

(57) **Zusammenfassung**

Die vorliegende Erfindung betrifft die Verwendung von 5'-Desoxy-5'-methylthioadenosin in hautkosmetischen Mitteln zur Verbesserung des Hautbildes, derartige Mittel und ein Verfahren zur kosmetischen Behandlung der Haut.

## Beschreibung

Die vorliegende Erfindung betrifft die Verwendung von 5'-Desoxy-5'-methylthioadenosin in hautkosmetischen Mitteln zur Verbesserung des Hautbildes, derartige Mittel und ein Verfahren zur kosmetischen Behandlung der Haut.

Die menschliche Haut unterliegt Veränderungen, die nicht zu den behandlungsbedürftigen Hauterkrankungen zählen, die aber dennoch von den Betroffenen als verbesserungsbedürftig empfunden werden. Dazu zählen z. B. Alterungsprozesse, wobei intrinsische Prozesse (chronoaging) und exogene Faktoren unterschieden werden können. Zusätzlich treten z. B. auch vorübergehende oder andauernde Veränderungen des Hautbildes auf, wie fettige oder trockene Haut und andere, deren genaue Ursachen sowie Faktoren, die sie beeinflussen, häufig nur unvollständig verstanden sind.

Zu den exogenen Faktoren zählen beispielsweise das Sonnenlicht oder künstliche Strahlungsquellen mit vergleichbarem Spektrum, sowie Verbindungen, die durch die Strahlung entstehen können, wie undefinierte reaktive, z. B. radikalische oder ionische Photoprodukte. Zu diesen Faktoren zählen aber auch schädliche oder reaktive Verbindungen wie Ozon, freie Radikale, beispielsweise das Hydroxylradikal, Singulettsauerstoff und andere reaktive Sauerstoff- oder Stickstoffverbindungen, Zigarettenrauch, natürliche und synthetische Toxine, und andere, die die natürliche Physiologie oder Morphologie der Haut stören. Durch den Einfluss dieser Faktoren kommt es beispielsweise zu direkten Schäden an der DNA der Hautzellen sowie den Kollagen-, Elastin- oder Glycosaminoglycanmolekülen der extrazellulären Matrix, die für die Festigkeit der Haut verantwortlich sind. Weiter kann es durch die schädlichen Einflüsse aber auch zu Schäden an den Zellen der Haut selbst kommen. Als Folge hiervon ist beispielsweise die Regenerationsfähigkeit der Haut verringert. Als weitere Folge kann es zu entzündlichen Reaktionen kommen, wobei unter Anderem immunregulatorische Verbindungen, wie Interleukine, Prostaglandine und Histamine eine Rolle spielen.

Die Folgen der Alterung sind Verdünnung der Haut, schwächere Verzahnung von Epidermis und Dermis, Reduktion der Zellzahl sowie der versorgenden Blutgefäße. Durch die Alterungsprozesse kommt es zur Ausbildung von feinen Linien und Falten, die Haut wird ledrig, gelblich und herabhängend, es treten Pigmentstörungen auf.

Es ist bekannt, dass Sonnenlichtexposition zu einer Vielzahl unerwünschter Effekte in der Haut führt: Erytheme, Photosensibilisierung und immunologische Veränderungen sind Beispiele für vorwiegend akute Reaktionen, während Photo-Alterung und Carcinogenese Langzeiteffekte darstellen. Weiterhin ist bekannt, dass nach Bestrahlung der Haut matrixabbauende Enzyme induziert werden und dass diese Induktion durch niedermolekulare Verbindungen, wie z. B. Retinsäuren, verringert werden kann. Ein weiterer Effekt der UV-Strahlung ist das Auftreten von Sonnenbrandzellen in der Haut. Dabei können nekrotische Prozesse auftreten, die eine inflammatorische Reaktion induzieren oder verstärken.

Die gleichen Faktoren wirken auch auf Haare, wo es ebenfalls zu einer Schädigung kommen kann. Die Haare werden spröde, weniger elastisch und glanzlos; die Oberflächenstruktur der Haare wird geschädigt.

An die Eigenschaften von kosmetischen Mitteln werden daher eine Vielzahl von Forderungen gestellt. So sollen sie unter anderem z. B. radikalfangend, entzündungshemmend oder feuchthaltend wirken, die Aktivität der matrixabbauenden Enzyme verhindern oder reduzieren und/oder die Neusynthese von Kollagen, Elastin und/oder Proteoglycanen regulieren.

Die WO-A-97/30713 beschreibt Zusammensetzungen zur Inhibierung von irreversiblen Zellveränderungen infolge von Stress, insbesondere Sauerstoffmangel, wie er beispielsweise ex vivo bei der Lagerung von Zellen, z. B. von Organen für die Transplantation und in vivo bei diversen operativen Eingriffen auftritt. Diese Zusammensetzungen enthalten wenigstens ein physiologisch verträgliches Purin-Derivat, vorzugsweise ein Purin-Ribosid. Neben Zusammensetzungen für die Lagerung von Zellen werden auch Nahrungsmittelzusammensetzungen beschrieben. Topische Anwendungen dieser Zusammensetzungen werden hingegen nicht beschrieben.

Die EP-A-0 526 866 beschreibt die Verwendung von Adenosin-Verbindungen, wie 5'-Desoxy-5'-methylthioadenosin zur Herstellung von pharmazeutischen Zusammensetzungen zur Behandlung von Ischämien.

Die EP-A-0 352 609 betrifft die Verwendung von Adenosinderivaten, wie 5'-Desoxy-5'-methylthioadenosin zur Herstellung von pharmazeutischen Zusammensetzungen mit immunstimulierender Wirkung.

Die EP-A-0 184 248 beschreibt Salze von 5'-Desoxy-5'-methylthioadenosin mit langkettigen Sulfonsäuren, die zentral und peripher gefäßerweiternd, gegen Verklumpung der Blutplättchen, entzündungshemmend, schmerzlindernd und fiebersenkend wirken.

Die US 4,373,097 beschreibt ein Verfahren zur Herstellung von Adenosin-Derivaten, wie 5'-Desoxy-5'-methylthioadenosin und deren entzündungshemmende, schmerzlindernde und fiebersenkende Wirkung.

R. Tritapepe und C. Pozzi beschreiben in Acta Therapeutica 15, 1989, Seiten 299-311 die Verwendung von 5'-Methylthioadenosin zur topischen Behandlung von Venenerkrankungen wie oberflächlicher Thrombophlebitis, akuter Venenthrombose, Varizen und Varizengeschwüren. Der Einsatz von 5'-Methylthioadenosin in der Kosmetik wird nicht beschrieben.

G. Pinto et al. beschreiben in Drug Invest 4 (3), Seiten 205-214, 1992, die topische Anwendung von 5'-Methylthioadenosin zur Behandlung der Symptomatik der chronischen Veneninsuffizienz, von Hämorrhoiden und oberflächlicher Phlebitis. Ein Einsatz in der Kosmetik wird wiederum nicht beschrieben.

M.A. Cerri et al. beschreiben im European Journal of Pharmacology, 232, 1993, Seiten 291-294 die Inhibierung der Cytokin-Produktion und der endothelialen Expression von Adhäsionsantigenen durch 5'-Methylthioadenosin.

Die EP-A-0 191 561 beschreibt ein Mittel gegen Karies oder Parodontose, das als Aktivkomponente Adenosin oder ein Adenosin-Derivat, wie 5'-Desoxy-5'-methylthioadenosin enthält.

Die EP-A-0 387 757 beschreibt die Verwendung von 5'-Desoxy-5'-methylthioadenosin zur Herstellung von oralen und topischen pharmazeutischen Zusammensetzungen zur Anregung des Haarwachstums bei Personen mit Glatzenbildung.

Die EP-A-0 387 756 betrifft die Verwendung von 5'-Desoxy-5'-methylthioadenosin in pharmazeutischen Zusammensetzungen, die sich zur Reduzierung einer übermäßigen Talgdrüsensekretion auf dem Kopf und damit verbundener Schuppenbildung und Hautjucken eignet. Diese Zusammensetzungen eignen sich für die orale und die topische Anwendung.

Die WO-A-94/14428 beschreibt ein Verfahren zur Verringerung des Haarwachstums bei Säugern, wobei man auf die Haut eine Zusammensetzung mit einer effektiven Menge einer Sulfhydryl-aktiven Verbindung aufträgt. Bei dieser Sulfhydryl-aktiven Verbindung kann es sich um 5'-Desoxy-5'-methylthioadenosin handeln.

Die WO 98/15276 beschreibt ein Verfahren zur Regulierung der Melaninproduktion in der Haut oder den Haaren, wobei man auf diese eine wirksame Menge einer pharmazeutischen Formulierung aufträgt, die ein Regulierungsmittel enthält. Bei diesem Regulierungsmittel kann es sich um einen Adenosin-2-rezeptorantagonisten, wie 5'-Desoxy-5'-methylthioadenosin handeln.

Der vorliegenden Erfindung liegt die Aufgabe zugrunde, ein Verfahren zur kosmetischen Behandlung der Haut und/oder der Haare zur Verfügung zu stellen, das sich zur Verbesserung des Hautbildes eignet. Das Verfahren soll sich insbesondere zur Behandlung von alterungsbedingten kosmetisch unerwünschten Veränderungen des Hautbildes eignen. Zudem soll möglichst auch Haut- und Haarschäden und/oder unerwünschten Veränderungen des Hautbildes vorgebeugt werden.

Überraschenderweise wurde nun gefunden, dass diese Aufgabe durch die Verwendung von 5'-Desoxy-5'-methylthioadenosin in hautkosmetischen Mitteln gelöst wird.

Unter "Verbesserung des Hautbildes" wird im Rahmen der vorliegenden Anmeldung jede positive oder auch vom Anwender positiv empfundene kosmetische Veränderung des Hautbildes verstanden. Die erfindungsgemäße Verwendung zur Behandlung von kosmetisch unerwünschten Veränderungen des Hautbildes betrifft die wesentlich von ästhetischen Überlegungen beim Anwender veranlasste Verwendung. Sie betrifft nicht oder nur untergeordnet eine wesentlich von medizinisch-therapeutischen Überlegungen veranlasste Verwendung. Die erfindungsgemäße Verwendung umfasst nicht die Anregung oder Verringerung des Haarwachstums sowie den Einsatz in topischen Mitteln zur Beschleunigung der Hautbräunung und zum Schutz der Haut gegen ultraviolette Strahlen (Lichtschutzmitteln) im Sinne einer physikalischen Abschirmung. Die erfindungsgemäße Verwendung umfasst jedoch die kosmetische Behandlung von Hautveränderungen, die unter Anderem auch durch Lichteinwirkung (mit)verursacht sein können.

Der Begriff "5'-Desoxy-5'-methylthioadenosin" bezeichnet im Rahmen der vorliegenden Erfindung Verbindungen der Formel I

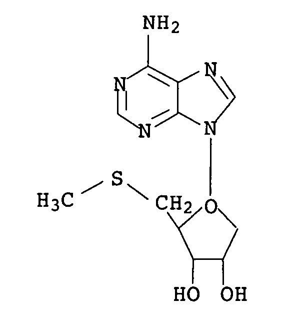

Die Verbindungen der Formel I werden auch als 5'-Methylthioadenosin, MTA, 7-[Tetrahydro-3,4-dihydroxy-5-(methylthiomethyl)-2-furyl]adenin und Vitamin L₂ bezeichnet.

Die Verbindungen der Formel I können in Form von reinen optischen Isomeren oder als Gemische eingesetzt werden.

Die erfindungsgemäße Verwendung umfasst auch kosmetisch akzeptable Salze. Bevorzugte kosmetisch akzeptable Salze des 5'-Desoxy-5'-methylthioadenosins sind Säureadditionssalze.

Zu den Säureadditionssalzen zählen Salze mit anorganischen Säuren, beispielsweise Phosphorsäure, oder mit Carbonsäuren, wie einfach oder mehrfach ungesättigten C₆-C₃₀-Monocarbonsäuren, wie Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, Palmetoleinsäure, Oleinsäure, Linolsäure, Linolensäure, Elaostearinsäure sowie Hydroxycarbonsäuren, wie Milchsäure, mehrwertigen Carbonsäuren, wie Citronensäure und Mischungen davon.

5'-Desoxy-5'-methylthioadenosin und die Salze davon eignen sich in vorteilhafter Weise zur Prävention und Behandlung von Schäden an keratinischen Oberflächen, d. h. Haut und Hautanhangsgebilden, wie z. B. Haaren, Nägeln etc., von Individuen, vorzugsweise Säugern, insbesondere Menschen, Nutz- oder Haustieren. Die Anwendung erfolgt dabei in Form von kosmetischen Mitteln wie Körperpflegemitteln, dekorativen Kosmetika etc., die in der Regel nicht verschreibungspflichtig sind. Davon zu unterscheiden ist die Anwendung in Form von Dermatika, worunter Medikamente zur Therapie von Erkrankungen der Haut (Dermatosen) verstanden werden.

Kosmetische Zubereitungen auf Basis von 5'-Desoxy-5'-methylthioadenosin und/oder Salzen davon bieten einen wirksamen Schutz vor oxidativen Prozessen, vor durch Strahlung oder reaktive Verbindungen hervorgerufenen Prozessen, vor Schäden, die durch derartige Prozesse direkt oder indirekt verursacht werden. Sie eignen sich vorteilhaft zur Behandlung von kosmetischen oder dermatologischen Veränderungen an Haut und Haar, wie z. B. der Hautalterung, dem Verlust der Hautfeuchtigkeit, dem Verlust der Hautelastizität, der Bildung von Falten oder Runzeln oder von Pigmentstörungen oder Altersflecken.

Weiterhin betrifft die vorliegende Erfindung die Verwendung zur kosmetischen Behandlung oder Vorbeugung unerwünschter Veränderungen des Hautbildes, wie Akne oder fettige Haut.

Besonders bevorzugt eignet sich 5'-Desoxy-5'-methylthioadenosin zur Verwendung in hautkosmetischen Mitteln zur Behandlung eines infolge Alterung veränderten Hautbildes. MTA dient dabei z. B. zur Verjüngung und/oder Revitalisierung der Haut. Vorteilhafterweise können dabei in der Regel auch energetisierende Effekte beobachtet werden. Insbesondere hat 5'-Desoxy-5'-methylthioadenosin einen positiven Effekt auf die Funktion der Mitochondrien. Überraschenderweise wird so oxidativen Schäden der Haut nicht nur vorgebeugt, sondern bereits vorhandene Schäden können, zumindest teilweise, beseitigt werden. Bei der erfindungsgemäßen Verwendung werden Verbesserungen beim Feuchtigkeitswert und/oder der Elastizität der Haut verzeichnet. Unter der Anwendung von 5'-Desoxy-5'-methylthioadenosin wird weiterhin die Neusynthese von Kollagen und/oder Elastin gesteigert. Dies führt im Allgemeinen zu einer, zumindest partiellen, Glättung von Falten, der Teint wirkt strahlender und frischer. In der Regel geht mit den genannten vorteilhaften Effekten auch ein positives subjektives Empfinden einher, insgesamt "jugendliche" Haut zu haben.

Formulierungen auf Basis von 5'-Desoxy-5'-methylthioadenosin eignen sich weiter vorteilhaft zur Behandlung, der Pflege und der Reinigung der Haut oder der Haare und kann als Schminkprodukt in der Kosmetik dienen. Die hautkosmetischen Mittel, in denen 5'-Desoxy-5'-methylthioadenosin verwendet wird, enthalten in der Regel wenigstens einen kosmetisch akzeptablen Träger. Geeignete Träger werden im Folgenden beschrieben. Sie enthalten bevorzugt 0,001 Gew.-% bis 30 Gew.-% des Wirkstoffs. Die Zusammensetzung richtet sich dabei z. B. nach den Penetrationseigenschaften der Wirksubstanz durch das Stratum Corneum und ihrer Fähigkeit in der Haut ein Depot zu bilden.

Nach einer bevorzugten Ausführungsform erfolgt die erfindungsgemäße Anwendung von 5'-Desoxy-5'-methylthioadenosin und/oder Salzen davon vorteilhaft durch regelmäßige Applikation, z. B. in Form einer kosmetischen Zubereitung, über eine Zeitspanne hinweg. Diese richtet sich nach dem angestrebten Ziel, d. h. die Zeitspanne kann sich über die Lebenszeit des Anwenders erstrecken, bevorzugt über eine Zeitspanne bis zu drei Monaten, besonders bevorzugt über eine Woche bis zwei Monate, wenn es das Ziel ist, ein Depot in der Haut aufzubauen. Für eine After-Sun-Anwendung gilt als Anwendungsdauer im Sinne der Erfindung die einmalige Applikation, bevorzugt jedoch eine Zeitspanne von mindestens einem Tag, besonders bevorzugt über drei Tage bis drei Monate, besonders bevorzugt über eine bis zwei Wochen.

Es ist im Sinne der Erfindung empfehlenswert, die kosmetische Zubereitung von 5'-Desoxy-5'-methylthioadenosin in einer Menge von 0,1 µg/cm² bis 2 mg/cm², zwischen 1-mal pro Woche und 4- bis 5-mal täglich, vorzugsweise 3-mal pro Woche bis 3-mal täglich, besonders bevorzugt 1- bis 2-mal täglich, topisch anzuwenden. Wirkstoffmengen und Anteile beziehen sich auf den aktiven Wirkstoff, so dass für Salze und Derivate eine entsprechende Umrechnung zu erfolgen hat.

Für After-Sun Anwendungen vorgesehene Darreichungsformen des 5'-Desoxy-5'-methylthioadenosins sowie gegebenenfalls zusätzlicher Wirkstoffe besitzen vorteilhaft Penetrationseigenschaften, die ein rasches Eindringen der Substanz in die Haut ermöglichen. Dagegen ist für Anwendungen mit "präkonditionierendem" Charakter eine schnelle Penetration in der Regel unwichtig, aber die Fähigkeit, in der Haut ein Depot aufzubauen, von Vorteil.

Überraschenderweise ist bei der erfindungsgemäßen Verwendung eine wirksame Behandlung aber auch Vorbeugung von vorzeitig gealterter Haut (z. B. Falten, Altersflecken, Teleangiektasien, Pigmentstörungen) und/oder vorzeitig gealterten Hautanhangsgebilden, strahlungsbedingten Hautschäden oder strahlungsbedingten negativen Veränderungen der Haut und/oder der Hautanhangsgebilde, umweltbedingten (Ozon, freie Radikale, Singulettsauerstoff, reaktive Sauerstoff- oder Stickstoffverbindungen, Zigarettenrauch, Toxine) Hautschäden oder umweltbedingten negativen Veränderungen der Haut und/oder der Hautanhangsgebilde, lichtempfindlichen, entzündlichen, erythematösen, allergischen oder autoimmunreaktiven Veränderungen der Haut und/oder der Hautanhangsgebilde (insbesondere Akne, fettige oder trockene Haut, Keratosen, Rosaceae, Dermatosen, atopisches Ekzem, seborrhoisches Ekzem, Photodermatosen, polymorphe Lichtdermatose), defizitären, sensitiven oder hypoaktiven Zuständen der Haut und/oder der Hautanhangsgebilde, Juckreiz, trockenen Hautzuständen und Hornschichtbarrierestörungen und/oder Haarausfall und verringertem Haarwachstum möglich.

Die erfindungsgemäße kosmetische Anwendung von 5'-Desoxy-5'-methylthioadenosin und/oder Salzen davon dient aber auch in überraschender und nicht vorhersehbarer Weise zur Beruhigung von empfindlicher und gereizter Haut. Sie dient weiterhin zur Regulation der Kollagen-, Hyaluronsäure-, Elastinsynthese, Stimulation der DNA-Synthese, insbesondere bei defizitären oder hypoaktiven Hautzuständen, Regulation der Transkription und Translation matrixabbauender Enzyme, insbesondere der Matrixmetalloproteinasen, Steigerung der Zellerneuerung und Regeneration der Haut, Steigerung der hauteigenen Schutz- und Reparaturmechanismen für DNA, Lipide und/oder Proteine. 5'-Desoxy-5'-methylthioadenosin kann auch in kosmetischen Mitteln zur Nachbehandlung bei chirurgischen Eingriffen verwendet werden, insbesondere um Hautreizungen entgegenzuwirken und die Regenerationsprozesse der verletzten Haut zu fördern.

Zur erfindungsgemäßen Verwendung werden die kosmetischen Zubereitungen in der für Kosmetika üblichen Weise auf die Haut und/oder die Haare in ausreichender Menge aufgebracht.

Beispielsweise wird 5'-Desoxy-5'-methylthioadenosin oder ein Salz davon in kosmetischen Mitteln zur Reinigung der Haut, wie Stückseifen, Toilettenseifen, Kernseifen, Transparentseifen, Luxusseifen, Deoseifen, Cremeseifen, Babyseifen, Hautschutzseifen, Abrasivseifen, Syndets, flüssige Seifen, pastöse Seifen, Schmierseifen, Waschpasten, flüssige Wasch-, Dusch- und Badepräparaten z.B. Waschlotionen, Duschbädern, Duschgelen, Schaumbädern, Cremeschaumbädern, Ölbädern, Badeextrakten, Scrubpräparaten, in-situ Produkten, Rasierschäumen, Rasierlotionen, Rasiercremes eingesetzt.

Weiterhin eignet sich 5'-Desoxy-5'-methylthioadenosin für hautkosmetische Zubereitungen wie W/O- oder O/W-Haut- und Körpercremes, Tag- und Nachtcremes, Augencremes, After-Sun-Produkte, Handpflegeprodukte, Gesichtcremes, Multiple Emulsionen, Gelees, Mikroemulsionen, Liposomenpräparate, Niosomenpräparate, Antifaltencremes, Gesichtsöle, Lipogele, Sportgele, Feuchthaltecremes, Bleichcremes, Vitamincremes, Hautlotionen, Pflegelotionen, Ampullen, After-Shave-Lotionen, Pre-Shaves, Feuchthaltelotionen, Cellulitecremes, Depigmentierungsmittel, Massagepräparate, Körperpuder, Gesichtswasser, Gesichtsmasken, Deodorantien, Antitranspirantien, Nose-Strips, Antiaknemittel, Repellents, Rasiermittel, Intimpflegemittel, Fußpflegemittel, Babypflegemittel und andere.

Außerdem kann 5'-Desoxy-5'-methylthioadenosin oder ein Salz davon in kosmetischen Mitteln für die Haarpflege wie Haarkuren, Haarlotionen, Haarspülungen, Haaremulsionen, Spitzenfluids, Egalisierungsmittel für Dauerwellen, Hot-Oil-Treatmentpräparate, Conditioner, Festigerlotionen, Shampoos, Haartönungs- und -färbemittel, Haarsprays, Fönlotionen, -festiger, Glanzsprays, Haarbrillantine, Haarstylingprodukte, Haarwasser und andere verwendet werden.

5'-Desoxy-5'-methylthioadenosin oder ein Salz davon eignen sich auch für die Anwendung in kosmetischen Zubereitungen für die dekorative Kosmetik, beispielsweise als Make-up, Puder, Rouge, Lidschatten, Kajalstifte, Eyeliner, Eyefoundationcreme, Lippenstifte, Augenbrauenstifte, Konturenstift, Abdeckstifte, Theaterschminke, Mascara, Wimperntönung, -färbung, Abschminkprodukte und andere.

Die kosmetischen oder hygienischen Zubereitungen können je nach Anwendungsgebiet als Spray (Pumpspray oder Aerosol), Schaum, Gel, Gelspray, Lotion, Creme, Mousse, Salbe, Suspensionen oder Pulver zubereitet werden.

Es ist auch vorteilhaft, 5'-Desoxy-5'-methylthioadenosin oder ein Salz davon gegebenenfalls mit weiteren Wirkstoffen in verkapseiter Form darzureichen, z. B. als Celluloseverkapselung, in Gelatine, mit Polyamiden, in Niosomen, Wachsmatrices, mit Cyclodextrinen oder liposomal verkapselt.

Die Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, Verdickungsmittel, oberflächenaktive Substanzen, Emulgatoren, weichmachende Substanzen, Avivagemittel, Fette, Öle, Wachse oder andere übliche Bestandteile einer kosmetischen oder dermatologischen Formulierung wie Alkohole, Polyole, Polymere, Schaumstabilisatoren, Lösungsvermittler, Elektrolyte, organische Säuren, organische Lösungsmittel oder Silikonderivate.

Die Zubereitungen können zusätzlich zu den genannten Wirkstoffen weitere Verbindungen enthalten die antioxidativ, als Radikalfänger, hautbefeuchtend oder -feuchthaltend, antierythematös, antientzündlich oder antiallergisch wirken, um deren Wirkung zu ergänzen oder zu verstärken. Insbesondere können diese Verbindungen ausgewählt werden aus der Gruppe der Vitamine, Pflanzenextrakte, α- und β-Hydroxysäuren, Ceramide, antiinflammatorischen, antimikrobiellen oder UV-filternden Substanzen, sowie deren Derivaten und Mischungen daraus.

Vorteilhaft sind die Antioxidantien ausgewählt unter Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivaten, Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butylund Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamöl, Sesamolin, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Antioxidantien können alleine oder in Form von Mischungen eingesetzt werden. Die Einsatzmenge der Antioxidantien in den Zubereitungen, alleine oder in Kombination, beträgt vorzugsweise 0,001 bis 30 Gew.-%, bezogen auf das Gesamtgewicht der Zubereitung.

Bevorzugt enthalten die Zubereitungen außerdem Substanzen, die UV-Strahlung im UV-B- und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl, und Mischungen davon. Geeignet sind weiterhin 4-Aminobenzoesäureester, wobei die Aminogruppe gegebenenfalls alkyliert oder alkoxiliert sein kann. Dazu zählt z. B. N,N-Dimethyl-4-aminobenzoesäureisooctylester. Geeignet sind weiterhin 2-Hydroxybenzoesäureester, wie z. B. der Isooctylester. Weitere geeignete UV-Filter sind 2,4,6-Trianilin-(o-carbo-2'-ethylhexyl--1'-oxy)-1,3,5-triazin,3-Imidazol-4-yl-acrylsäure und ihr Ethylester, Menthyl-o-aminobenzoat, Glyceryl-p-aminobenzoat, 2,2'-Dihydroxy-4-methoxybenzophenon (Dioxybenzone), 2-Hydroxy-4-methoxy-4-methylbenzophenon (Mexenon®), Triethanolaminsalicylat, Dimethoxyphenylglyoxalsäure, 3-(4'Sulfo)benzyliden-bornan-2-on und seine Salze, 2,2',4,4'-Tetrahydroxybenzophenon, 2,2'-Methylen-bis [6(2H-benzotriazol-2-yl-4-(1,1,3,3,-tetramethylbutyl)phenol], 2,2'-(1,4-Phenylen)-bis-1H-benzimidazol-4,6-disulfonsäure und sein Na-Salz, 2,4-bis-[4-(2-Ethylhexyloxy)-2-hydroxy]phenyl-6-(4-methoxyphenyl)-(1,3,5)-triazin, 3-(4-Methylbenzyliden)-campher, 4-Bis(polyethoxy)paraaminobenzoesäurepolyethoxyethylester, 2,4-Dihydroxybenzophenon und/oder 2,2'-Dihydroxy-4,4'-dimethoxybenzophenon-5,5'-dinatriumsulfonat.

Die Liste der genannten UV-Filter, die in Kombination mit den erfindungsgemäßen Wirkstoffkombinationen verwendet werden können, soll selbstverständlich nicht limitierend sein.

Die Gesamtmenge der Filtersubstanzen beträgt in der Regel 0,1 Gew.-% bis 30 Gew.-%, vorzugsweise 0,5 bis 15 Gew.-%, insbesondere 1 bis 10 Gew.-%, bezogen auf das Gesamtgewicht der Zubereitungen, um kosmetische Zubereitungen zur Verfügung zu stellen, die die Haut vor dem gesamten Bereich der ultravioletten Strahlung schützen.

Wird eine Lipidphase eingesetzt, so ist diese vorzugsweise ausgewählt unter Mineralölen, Mineralwachsen, verzweigten und/oder unverzweigten Kohlenwasserstoffen und -wachsen, Triglyceriden gesättigter und/oder ungesättigter, verzweigter und/oder unverzweigter C₈-C₂₄-Alkancarbonsäuren; synthetischen, halbsynthetischen oder natürlichen Ölen wie Olivenöl, Palmöl, Mandelöl oder Mischungen; Ölen, Fetten oder Wachsen; Estern aus gesättigten und/oder ungesättigten, verzweigten und/oder unverzweigten C₃-C₃₀-Alkancarbonsäuren und gesättigten und/oder ungesättigten, verzweigten und/oder unverzweigten C₃-C₃₀-Alkoholen, Estern aus aromatischen Carbonsäuren und gesättigten und/oder ungesättigten, verzweigten und/oder unverzweigten C₃-C₃₀-Alkoholen, beispielhaft Isopropylmyristat, Isopropylstearat, Hexyldecylstearat, Oleyloleat; synthetischen, halbsynthetischen und natürlichen Gemischen der zuvor genannten Ester, wie Jojobaöl, Alkylbenzoaten oder Silikonölen wie z. B. Cyclomethicon, Dimethylpolysiloxan, Diethylpolysiloxan, Octamethylcyclotetrasiloxan sowie Mischungen daraus oder Dialkylether.

Wird eine wässrige Phase eingesetzt, so enthält sie gegebenenfalls zusätzlich ein wassermischbares Lösungsmittel, wie C₁-C₁₀-, bevorzugt C₁-C₅-Alkohole, -Diole oder -Polyole, sowie deren Ether, vorzugsweise Ethanol, Isopropanol, Propylenglykol, Glycerin, Ethylenglykolmonoethylether etc.

Als Emulgatoren kommen vorzugsweise bekannte W/O- und O/W-Emulgatoren wie Polyglycerinester, Sorbitanester oder teilveresterte Glyceride in Betracht.

Als geeignete Lösungsvermittler sind insbesondere ethoxylierte Sorbitanester, ethoxylierte Lanolinalkohole und ethoxyliertes Rizinusöl zu nennen.

Übliche native und synthetische Verdickungsmittel bzw. Gelbildner in den Formulierungen sind vernetzte Polyacrylsäuren und deren Derivate, Polysaccharide wie Xanthan Gum oder Alginate, Carboxymethylcellulose oder Hydroxycarboxymethylcellulose, Hydrokolloide wie Gummi Arabicum oder Motmorillonitmineralien wie Bentonite oder Fettalkohole, Polyvinylalkohol und Polyvinylpyrrolidon.

Als Treibmittel für Aerosole kommen die üblichen Treibmittel in Frage, beispielhaft Propan, Butan, Pentan, Dimethylether und andere.

Ein weiterer Gegenstand der Erfindung ist ein hautkosmetisches Mittel, ausgenommen Mittel zur Bräunungsbeschleunigung und daraus resultierenden Lichtschutz, enthaltend
I) 5'-Desoxy-5'-methylthioadenosin und/oder ein kosmetisch akzeptables Salz davon,
II) gegebenenfalls wenigstens einen weiteren kosmetischen Wirkstoff, und
III) einen kosmetisch akzeptablen Träger.

Unter einem ausgenommenen Lichtschutzmittel wird dabei ein Mittel verstanden, das unmittelbar bevor die Haut und/oder die Haare dem Licht ausgesetzt werden, aufgetragen wird, um eine unmittelbare Schädigung der Haut und/oder der Haare, wie sie insbesondere durch den UV-B-Anteil im Sonnenlicht verursacht wird, zu vermeiden.

Vorzugsweise enthalten die Mittel die Komponenten I in einer Menge von 0,001 bis 30 Gew.-%, insbesondere 0,01 bis 25 Gew.-%, bezogen auf das Gesamtgewicht des Mittels.

Bezüglich geeigneter Komponenten II) und III) wird auf das vorher Gesagte Bezug genommen.

Ein weiterer Gegenstand der vorliegenden Erfindung sind Mittel in Form einer Handelspackung mit wenigstens einem Mittel auf Basis von 5'-Desoxy-5'-methylthioadenosin gegebenenfalls zusammen mit Instruktionen für die kosmetische Anwendung.

Es versteht sich, dass erfindungsgemäße Handelspackungen auch weitere Präparate, insbesondere wirkstoffhaltige Formulierungen, sowie umfassende, auch über den vorstehend genannten Inhalt hinausgehende Instruktionen enthalten können.

Ein weiterer Gegenstand der Erfindung ist ein Verfahren zur kosmetischen Behandlung der Haut, bei dem man auf die Haut eine kosmetische Zubereitung, enthaltend 5'-Desoxy-5'-methylthioadenosin und/oder ein kosmetisch akzeptables Salz davon, in einer zur Verbesserung des Hautbildes wirksamen Menge aufträgt.

Die Erfindung wird anhand der nachfolgenden, nicht einschränkenden Beispiele näher erläutert.

### Beispiele kosmetischer Zubereitungen

| Formulierungstyp | Anwendungsbereich | Beispiel No |
|---|---|---|
| O/W-Emulsion | Soft Skin Lotion | 1 |
| W/O-Emulsion | schützende Handcreme | 2 |
| | Sonnenpflege-Lotion | 3 |
| Multiple Emulsion | W/O/W-Emulsion | 4 |
| Mikroemulsion | Microemulsion | 5 |
| Hydrophiles Gel | Liposomengel | 6 |
| Lipophiles Gel | Blunted Oil Gel | 7 |
| | Oil Gel | 8 |
| Stiftformulierung | pflegender Sonnenschutz- | 9 |
| | Lippenstift | |
| Wässrige Kosmetika | kühlender Body Splash | 10 |
| Dekorative Kosmetik | Make up | 11 |
| | Flüssig-Make up | 12 |
| Öle | Sonnenpflege-Öl | 13 |
| Körperreinigungsmittel | Gesichts-Tiefenreiniger | 14 |
| Haarnachbehandlungsmittel zum Ausspülen | Conditioner | 15 |
| Haarnachbehandlungsmittel zum Nichtausspülen | Haarwachs | 16 |
| | Anti-Schuppen Haartonikum | 17 |
| Aerosol | Fuß-Deospray | 18 |
| | Haarspray | 19 |

### Beispiele kosmetischer Zubereitungen

### Beispiel 1: Soft Skin Fluid

| | Gew.-% |
|---|---|
| Ceteareth-6 (Umsetzungsprodukt aus Fettalkohol und Ethylenoxid) und Stearylalkohol | 2,50 |
| Ceteareth-25 (Umsetzungsprodukt aus Fettalkohol und Ethylenoxid) | 2,50 |
| Hydrierte Kokosfett-Glyceride | 1,50 |
| Polyethylenglykol (40 EO-Einheiten)-Dodecylglycolcopolymer | 3,00 |
| Dimethicon | 3,00 |
| Phenethyldimethicon | 2,00 |
| Cyclomethicon | 1,00 |
| Cetearyloctanoat | 5,00 |
| Avocadoöl | 1,00 |
| Süßmandelöl | 2,00 |
| Weizenkeimöl | 0,80 |
| Panthenol USP | 1,00 |
| Phytantriol | 0,20 |
| Tocopherylacetat | 0,30 |
| Propylene Glycol | 5,00 |
| Parfum | q.s. |
| Konservierungsstoffe | q.s. |
| 5'-Desoxy-5'-methylthioadenosin | q.s. |
| Wasser | 69,20 |

### Beispiel 2: Schützende Handcreme

| | Gew.-% |
|---|---|
| Cetearylalkohol | 1,00 |
| Glycerylstearat | 1,50 |
| Stearylalkohol | 1,50 |
| Cetylpalmitat | 2,00 |
| Tocopherylacetat | 0,50 |
| Dimethicon | 8,00 |
| Ceteareth-6 (Umsetzungsprodukt aus Fettalkohol und Ethylenoxid) und Stearylalkohol | 3,00 |
| Octylmethoxycinnamat | 5,00 |
| Propylenglycol | 8,00 |
| Panthenol | 1,00 |
| Nachtkerzenöl | 3,00 |
| ethoxyliertes hydriertes Ricinusöl (7 EO) | 6,00 |
| Glyceryloleat | 1,00 |
| Phenethyldimethicon | 3,00 |
| Bienenwachs | 1,50 |
| Johannisbrot-Gummi | 0,80 |
| Silkpowder | 0,80 |
| Konservierungsstoffe | q.s. |
| Parfum | q.s. |
| Borax | 0,10 |
| 5'-Desoxy-5'-methylthioadenosin | q.s. |
| Wasser | 52,30 |

### Beispiel 3: Multiple Emulsion

| | Gew.-% |
|---|---|
| Mineralöl | 7,50 |
| Cetearyloctanoat | 2,50 |
| Aluminiumstearat | 0,25 |
| Magnesiumstearat | 0,25 |
| Mikrokristallines Wax H | 0,50 |
| Cetearylalkohol | 1,00 |
| Lanolinalkohol | 1,50 |
| Mineralalkohol und Lanolinalkohol | 1,50 |
| ethoxiliertes hydriertes Rininusöl (7 EO) | 0,75 |
| Polyethylenglykol (40 EO)-Dodecyl-glykolcopolymer | 2,00 |
| Ceteareth-6 (Umsetzungsprodukt aus Fettalkohol und Ethylenoxid) und Stearylalkohol | 2,00 |
| Ceteareth-25 (Umsetzungsprodukt aus Fettalkohol und Ethylenoxid) | 2,00 |
| Trilauret-4 Phosphat | 1,00 |
| Hydroxyethylcellulose | 0,20 |
| Propylenglycol | 7,50 |
| Magnesiumsulfat | 0,25 |
| 5'-Desoxy-5'-methylthioadenosin | q.s. |
| Wasser | 69,30 |

### Beispiel 4: Microemulsion

| | Gew.-% |
|---|---|
| Ceteareth-25 (Umsetzungsprodukt aus Fettalkohol | |
| und Ethylenoxid) | 13,00 |
| Glycerylcocoat (7 EO) | 20,00 |
| Octyldodecanol | 5,00 |
| Konservierungsstoffe | q.s. |
| 5'-Desoxy-5'-methylthioadenosin | q.s. |
| Wasser | 62,00 |

### Beispiel 5: Liposomengel

| | Gew.-% |
|---|---|
| ethoxiliertes hydriertes Ricinusöl (40 EO) | 1,00 |
| Bisabolol rac. | 0,10 |
| Propylenglykol | 8,00 |
| Panthenol | 0,50 |
| Wasser und Tocopherylacetat und Polysorbate 80 und Capryl/Caprin-Triglycerid und Lecithin | 3,00 |
| Konservierungsstoffe | q.s. |
| Parfum | q.s. |
| Carbomer | 0,50 |
| 5'-Desoxy-5'-methylthioadenosin | q.s. |
| Triethanolamin | 0,70 |
| Wasser | 86,30 |

### Beispiel 6: Blunted Oil Gel

| | Gew.-% |
|---|---|
| Siliciumdioxid | 5,00 |
| Dimethicon | 10,00 |
| Cetearyloctanoat | 40,00 |
| Capryl/Caprin- Triglycerid | 8,00 |
| Phenethyldimethicon | 2,00 |
| Mineralöl | 28,50 |
| Süßmandelöl | 5,00 |
| Phytantriol | 0,30 |
| 5'-Desoxy-5'-methylthioadenosin | q.s. |
| Tocopherol | 0,50 |
| Parfum | 1,00 |

### Beispiel 7: Öl Gel

| | Gew.-% |
|---|---|
| Siliciumdioxid | 5,00 |
| Dimethicon | 10,00 |
| Cetearyloctanoat | 30,00 |
| Capryl/Caprin- Triglycerid | 10,00 |
| Isopropylmyristat | 5,00 |
| Phenethyldimethicon | 5,00 |
| Mineralöl | 28,20 |
| Jojobaöl | 5,00 |
| Phytantriol | 0,30 |
| 5'-Desoxy-5'-methylthioadenosin | q.s. |
| Tocopherol | 0,50 |
| Parfum | 1,00 |

### Beispiel 8: Kühlender Body Splash

| | Gew.-% |
|---|---|
| Ethoxyliertes hydriertes Ricinusöl (40 EO) | 2,00 |
| Menthyllactat | 0,20 |
| Alkohol | 5,00 |
| Glycerylcocoat (7 EO) | 2,00 |
| Witch Hazel | 5,00 |
| Allantoin | 0,10 |
| Bisabolol rac. | 0,20 |
| Propylenglycol | 5,00 |
| Panthenol USP | 0,50 |
| Milchsäure (80%ig) | 0,20 |
| 5'-Desoxy-5'-methylthioadenosin | q.s. |
| Parfum | q.s. |
| Wasser | 79,80 |

### Beispiel 9: Make-up

| | Gew.-% |
|---|---|
| Ceteareth-6 (Umsetzungsprodukt aus Fettalkohol und Ethylenoxid) und Stearylalkohol | 9,00 |
| Dimethicon | 5,00 |
| Cetearyloctanoat | 8,00 |
| Macadamianußöl | 5,00 |
| Propylenglycol | 5,00 |
| Wasser | 53,00 |
| Sicovit White E 171 | 8,00 |
| Sicomet Brown 70 13E 3717 | 2,00 |
| 5'-Desoxy-5'-methylthioadenosin | q.s. |
| Parfum | q.s. |
| 3-Benzophenon | 5,00 |

### Beispiel 10: Flüssig-Make-up

| | Gew.-% |
|---|---|
| Ceteareth-6 (Umsetzungsprodukt aus Fettalkohol und Ethylenoxid) und Stearylalkohol | 7,00 |
| Ceteareth-25 | 5,00 |
| Dimethicon | 5,00 |
| Cetearyloctanoat | 8,00 |
| Macadamianußöl | 5,00 |
| Propylenglycol | 5,00 |
| Wasser | 53,00 |
| Sicovit Weiss E 171 | 8,00 |
| Sicomet Braun 70 13E 3717 | 1,00 |
| 5'-Desoxy-5'-methylthioadenosin | q.s. |
| Parfum | q.s. |
| 3-Benzophenon | 5,00 |

### Beispiel 11: Gesichts-Tiefenreiniger

| | Gew.-% |
|---|---|
| Wasser | 65,60 |
| Cocoamidopropylbetain | 5,00 |
| Natrium-Cocoat, hydrolysiertes tierisches Protein | 8,00 |
| Ethoxyliertes hydriertes Ricinusöl (40 EO) | 2,00 |
| Polyquaternium-44 | 7,70 |
| Bisabolol rac. | 0,20 |
| Panthenol | 1,00 |
| Parfum | 0,50 |
| Hydroxyethylcellulose | 2,00 |
| 5'-Desoxy-5'-methylthioadenosin | q.s. |
| Propylenglycol | 5,00 |
| Jojobawachs | 3,00 |

### Beispiel 12: Conditioner

| | Gew.-% |
|---|---|
| Ceteareth-6 und Stearylalkohol | 2,00 |
| Ceteareth-25 (Umsetzungsprodukt aus Fettalkohol und Ethylenoxid) | 1,00 |
| Cetearyloctanoat | 6,00 |
| Ceteareth-3 | 2,00 |
| Cetearylalkohol | 6,00 |
| Phytantriol | 1,00 |
| Propylenglykol | 5,00 |
| Polyquaternium-11 | 5,00 |
| Panthenol | 1,00 |
| Retinylacetat | 0,50 |
| Parfum | q.s. |
| 5'-Desoxy-5'-methylthioadenosin | q.s. |
| Konservierungsstoffe | q.s. |
| Wasser | 70,50 |

### Beispiel 13: Haarwachs

| | Gew.-% |
|---|---|
| Polyethylenglycol-6 | 30,00 |
| Polyethylenglycol-75 | 45,00 |
| flüssiges Paraffinöl | 0,50 |
| Ethoxyliertes hydriertes Ricinusöl (40 EO) | 1,00 |
| Glycerin | 15,00 |
| 3-Benzophenon | 2,00 |
| Phytantriol | 0,10 |
| 5'-Desoxy-5'-methylthioadenosin | q.s. |
| Parfum | q.s. |
| Wasser | 6,40 |

### Beispiel 14: Anti-Schuppen Haartonikum

| | Gew.-% |
|---|---|
| Alkohol | 45,00 |
| Aloe Vera (10fach Konz.) | 1,00 |
| Panthenol | 1,00 |
| Tocopherylacetat | 0,50 |
| Ethoxyliertes hydriertes Ricinusöl (40 EO) | 0,50 |
| Allantoin | 0,10 |
| Hydrolysiertes tierisches Protein | 1,50 |
| 1-(4-Chlorphenoxy)-1-(1H-imidazolyl)-3,3-dimethyl2-butanon | 0,30 |
| Parfum | 0,10 |
| 5'-Desoxy-5'-methylthioadenosin | q.s. |
| Wasser | 50,00 |

### Beispiel 15: Fuß-Deospray

| | Gew.-% |
|---|---|
| Ethoxyliertes hydriertes Ricinusöl (40 EO) | 0,80 |
| Alkohol | 20,00 |
| Farnesol | 0,12 |
| Menthyllactat | 0,08 |
| 1,2-Propylenglykol | 3,20 |
| 4-Benzophenon | 1,20 |
| Glycerylcocoat (7 EO) | 0,80 |
| Parfum | q.s. |
| 5'-Desoxy-5'-methylthioadenosin | q.s. |
| Wasser | 13,80 |
| Butan | 60,00 |

### Beispiel 16: Haarspray

| | Gew.-% |
|---|---|
| Aminomethylpropanol | 0,40 |
| Dimethiconcopolyol | 0,030 |
| Alkohol | 43,67 |
| Pentan | 14,20 |
| Acrylat/Acrylamid-Copolymer | 3,40 |
| Parfum | q.s. |
| 5'-Desoxy-5'-methylthioadenosin | q.s. |
| Butane | 2,40 |
| Isobutan | 35,90 |

## Patentansprüche

1. Verwendung von 5'-Desoxy-5'-methylthioadenosin in hautkosmetischen Mitteln zur Verbesserung des Hautbildes.

2. Verwendung nach Anspruch 1, wobei das Hautbild infolge einer Alterung verändert ist.

3. Verwendung nach einem der vorhergehenden Ansprüche, wobei das Mittel wenigstens einen kosmetisch akzeptablen Träger enthält.

4. Verwendung nach einem der vorhergehenden Ansprüche, wobei das Mittel 5'-Desoxy-5'-methylthioadenosin in einer Menge von 0,001 bis 30 Gew.-% bezogen auf das Gesamtgewicht des Mittels enthält.

5. Hautkosmetisches Mittel, ausgenommen Mittel zur Bräunungsbeschleunigung, enthaltend
I) 5'-Desoxy-5'-methylthioadenosin und/oder ein kosmetisch akzeptables Salz davon,
II) gegebenenfalls wenigstens einen weiteren kosmetischen Wirkstoff, und
III)einen kosmetisch akzeptablen Träger.

6. Mittel nach Anspruch 5, das die Komponente I in einer Menge von 0,001 bis 30 Gew.-% bezogen auf das Gesamtgewicht des Mittels enthält.

7. Mittel nach einem der Ansprüche 5 oder 6 in Form einer Handelspackung, gegebenenfalls zusammen mit Instruktionen zur kosmetischen Anwendung.

8. Verfahren zur kosmetischen Behandlung der Haut, bei dem man auf die Haut eine kosmetische Zubereitung, enthaltend 5'-Desoxy-5'-methylthioadenosin und/oder ein kosmetisch akzeptables Salz davon, in einer zur Verbesserung des Hautbildes wirksamen Menge aufträgt.
